Europäisches Patentamt

European Patent Office

Office européen des brevets

(11) Numéro de publication: **0 076 180**

**A2**

# (12) DEMANDE DE BREVET EUROPEEN

(21) Numéro de dépôt: **82401614.1**

(22) Date de dépôt: **01.09.82**

(51) Int. Cl.³: **A 61 B 6/02**
**G 03 B 41/16**

(30) Priorité: **25.09.81 FR 8118121**

(43) Date de publication de la demande:
**06.04.83 Bulletin 83/14**

(84) Etats contractants désignés:
**AT BE CH DE GB IT LI LU NL SE**

(71) Demandeur: **Coucy, René**
**100 rue Nationale Bleriot Plage**
**Pas-de-Calais(FR)**

(72) Inventeur: **Coucy, René**
**100 rue Nationale Bleriot Plage**
**Pas-de-Calais(FR)**

(74) Mandataire: **Bonnetat, Christian et al,**
**Cabinet PROPI Conseils 23 rue de Léningrad**
**F-75008 Paris(FR)**

(54) **Procédé et dispositif pour la réalisation d'images tomographiques.**

(57) Selon l'invention, on forme, à partir du signal reçu par un capteur (7), un trait lumineux (9 ou 9, 11) dont l'intensité instantanée est uniforme le long dudit trait et correspond à la quantité instantanée de rayonnement R reçue par ledit capteur (7), et on fait effectuer audit trait lumineux (9) un mouvement semblable et synchrone de celui du faisceau d'exploration R de sorte à balayer une pluralité de fois une zone d'enregistrement suivant une pluralité de directions tournant avec la rotation dudit bâti (1).

Applications médicales.

Fig. 1

EP 0 076 180 A2

0076180

# Procédé et dispositif pour la réalisation d'images tomographiques.

La présente invention concerne un procédé et un dispositif pour la réalisation d'images tomographiques. Elle concerne les appareils d'analyse par balayage, notamment utilisés en médecine et désignés généralement sous les noms de "scanner", scanographes, tomodensitomètres, etc...

On sait que ces appareils scanographiques connus comportent un bâti rotatif tournant autour de l'objet ou du corps à observer, une source de rayonnements solidaire en rotation dudit bâti et au moins un capteur recevant le faisceau d'exploration émis par ladite source après la traversée dudit objet ou dudit corps à observer, ledit faisceau d'exploration effectuant, outre un mouvement de rotation correspondant à celui dudit bâti, un mouvement de balayage alternatif dans un plan orthogonal à l'axe de rotation de ce bâti.

Dans ces appareils connus, l'exploitation des signaux reçus par ledit capteur, en vue de former l'image tomographique, est effectuée par une calculatrice électronique. L'image tomographique obtenue est très précise, mais en revanche, le coût des appareils connus est très élevé, le prix de la calculatrice étant au moins égal au prix du reste de l'appareil.

La présente invention a pour objet un procédé et un dispositif pour la réalisation d'images tomographiques permettant d'éviter l'utilisation d'une calculatrice électronique et donc d'abaisser fortement le coût des appareils scanographiques, sans pour autant en diminuer les performances.

On remarquera que, par le brevet français 74 14930 (2 269 074), on connaît déjà un appareil scanographique dans lequel l'image est formée par un dispositif électronique, ne comportant aucune calculatrice. Cependant, la présente invention met en oeuvre un principe entièrement différent et d'une grande simplicité, permettant un abaissement important du coût de l'appareil scanographique.

A cette fin, selon l'invention, le procédé pour la réalisation d'images tomographiques au moyen d'un appareil scanographique comportant un bâti rotatif tournant autour de l'objet ou du corps à observer, une source de rayonnements solidaire en rotation dudit bâti et au moins un capteur recevant le faisceau d'exploration émis par ladite source après la traversée dudit objet ou dudit corps à observer, ledit faisceau d'exploration effectuant, outre un mouvement de rotation correspondant à celui dudit bâti, un mouvement de balayage alternatif dans un plan orthogonal à l'axe de rotation de ce bâti, est remarquable en ce que l'on forme, à partir du signal reçu par ledit capteur, un trait lumineux dont l'intensité instantanée est uniforme le long dudit trait et correspond à la quantité instantanée de rayonnement reçue par ledit capteur et en ce que l'on fait effectuer audit trait lumineux un mouvement semblable et synchrone de celui du faisceau d'exploration, de sorte à balayer une pluralité de fois une zone d'enregistrement suivant une pluralité de directions tournant avec la rotation dudit bâti.

Ainsi, sur ladite zone d'enregistrement, on obtient par superposition d'une pluralité d'images du trait lumineux, l'image scanographique du corps observé.

Pour la mise en oeuvre du procédé selon l'invention, l'appareil scanographique comportant un bâti rotatif tournant autour de l'objet ou du corps à observer, une source de rayonnements solidaire en rotation dudit bâti et au moins un capteur recevant le faisceau d'exploration émis par ladite source après la traversée dudit objet ou dudit corps à observer, ledit faisceau d'exploration effectuant, outre un mouvement de rotation correspondant à celui dudit bâti, un mouvement de balayage alternatif dans un plan orthogonal à l'axe de rotation de ce bâti, est remarquable en ce qu'il comporte un dispositif lumineux linéaire dont l'intensité est uniforme le long dudit dispositif et correspond à la quantité instantanée de rayonnement reçue par le capteur, et des moyens de couplage imposant audit dispositif lumineux linéaire un

mouvement synchrone de celui du faisceau d'exploration, de sorte que ce dispositif lumineux linéaire balaie une pluralité de fois une zone d'enregistrement suivant une pluralité de directions tournant avec la rotation dudit bâti.

Dans un mode avantageux de réalisation, le dispositif lumineux linéaire est constitué, au moins en partie, par un alignement d'éléments électroluminescents, tels que des diodes. A un instant donné, tous les éléments électroluminescents produisent un éclairement identique. Cet éclairement peut être significatif de la quantité de rayonnement reçue par le capteur.

Pour obtenir une bonne définition d'image, il peut être avantageux d'utiliser un écran pourvu d'une fente mince, éclairé par ledit alignement d'éléments électroluminescents. La longueur du trait lumineux doit dans tous les cas rester inférieure au diamètre du faisceau d'exploration.

En variante, l'éclairement du trait lumineux est constant et l'on prévoit, entre le dispositif lumineux linéaire et la zone d'enregistrement, un filtre à transmission variable commandée, dont le facteur de transmission instantané est représentatif de la quantité de rayonnement reçue par le capteur.

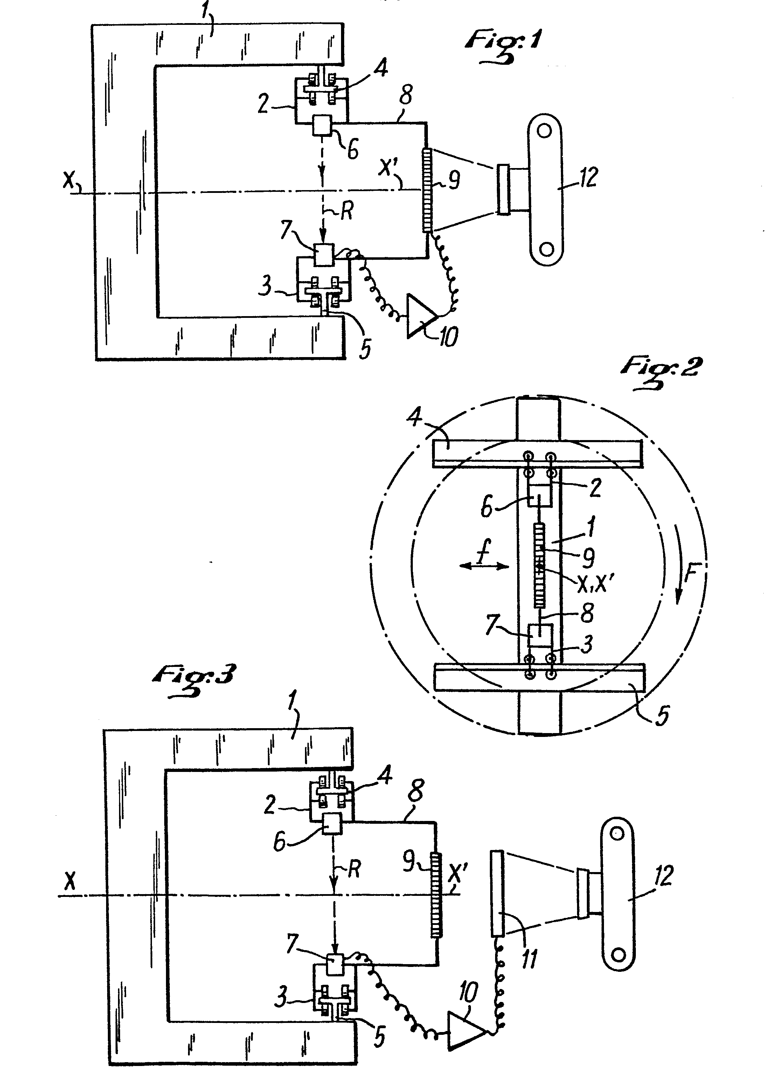

Dans le cas où le balayage alternatif du faisceau d'exploration est obtenu par voie mécanique (ce qui est représenté dans les exemples de réalisation décrits ci-après en regard des figures), le dispositif lumineux linéaire peut être mécaniquement rendu solidaire de la pièce en mouvement produisant ce balayage. Dans le cas où le balayage alternatif du faisceau d'exploration est obtenu par voie électrique (ce qui n'est pas représenté sur les dessins annexés, mais fait néanmoins partie de l'invention), le mouvement du dispositif lumineux est synchronisé électriquement sur celui dudit faisceau d'exploration. Bien entendu, dans son balayage alternatif, le faisceau peut se déplacer soit par translation parallèlement à lui-même (comme sur les figures 1 à 3), soit par pivotement.

4

La zone d'enregistrement peut être constituée de tout 0076180
support rémanent approprié et notamment par tout film
photographique. Dans ce dernier cas, le film photographique
est introduit dans un appareil photographique pointé sur le
dispositif lumineux linéaire et l'obturateur de l'appareil
reste ouvert pendant la totalité de l'exploration du corps
ou objet dont on désire des tomographies.

Bien entendu, le procédé et le dispositif selon l'invention
peuvent être mis en oeuvre aussi bien dans le cas où se
produisent une pluralité de balayages du faisceau d'exploration
pendant une rotation du bâti, que dans le cas où le bâti
décrit plusieurs rotation pendant un balayage du faisceau
d'exploration.

Les figures du dessin annexé feront bien comprendre comment
l'invention peut être réalisée.

La figure 1 est une vue latérale schématique d'un exemple
de réalisation d'un appareil scanographique selon
l'invention.

La figure 2 est une vue de face de l'appareil de la figure
1.

La figure 3 illustre, en vue semblable à la figure 1, la
variante de réalisation de l'appareil selon l'invention.

L'appareil scanographique selon l'invention, montré par ces
figures, comporte un bâti 1, par exemple en forme de C,
monté rotatif autour d'un axe médian X-X'. Sur le bâti
rotatif 1 sont montés, en regard l'un de l'autre, des
chariots 2 et 3 pouvant respectivement coulisser sur des
rails 4 et 5. Les rails 4 et 5 sont parallèles entre eux et
déterminent un plan ortogonal à l'axe X-X'.

Le chariot 2 porte une source de rayonnement 6, tandis que
le chariot 3 porte un capteur de rayonnement 7. Par une

liaison mécanique 8, la source 6 et le capteur 7 sont solidarisés l'un de l'autre, de façon à rester constamment en regard, lorsque le bâti 1 tourne autour de l'axe X-X' (flèche F) et que les chariots 2 et 3 effectuent un mouvement de va-et-vient (flèches f) le long des rails 4 et 5. Sur les figures, les moyens d'entraînement du bâti 1 et des chariots 2 et 3 ne sont pas représentés à des fins de clarté. De même, on n'a pas représenté le corps à observer se trouvant sur le trajet R du faisceau allant de la source 6 au capteur 7.

Une barrette lumineuse 9 est rendue solidaire de la liaison mécanique 8 de façon que ladite barrette soit parallèle au plan défini par les rails 4 et 5, dans lequel se trouve le rayonnement R émis par la source 6 et reçu par le capteur 7. Ainsi, au cours du mouvement résultant de la rotation F du bâti et du mouvement de va-et-vient f des chariots 2 et 3 le long des rails 4 et 5, la barrette 9 décrit un mouvement synchrone de celui du faisceau d'exploration R émis par la source 6 en lui restant constamment parallèle.

Dans le mode de réalisation des figures 1 et 2, la barrette 9 est électriquement reliée au capteur 7, par exemple par l'intermédiaire d'un amplificateur 10, de façon qu'à un instant donné toutes les diodes de la barrette 9 présentent un éclairement identique fonction de l'intensité du faisceau d'exploration R reçu à cet instant par le capteur 7. Dans le mode de réalisation de la figure 3, l'éclairement de la barrette 9 est égal et constant, mais on prévoit un filtre 11 à variations de transmission commandées (par exemple du type polarisant à cristaux liquides) dont l'entrée de commande est reliée au capteur 7, par exemple par l'intermédiaire de l'amplificateur 10.

Dans les deux cas, si l'on place un appareil photographique 12 observant la barrette 9 soit directement (figures 1 et 2) soit à travers le filtre 11, on conçoit aisément que la pellicule photographique de l'appareil 12 constitue une zone d'enregistrement balayée par une pluralité de fois suivant une pluralité de directions tournant avec le bâti 1 par le trait lumineux formé par la barrette 9, éventuellement associée au filtre 11. Comme l'intensité de ce trait lumineux est une fonction de l'intensité du faisceau d'exploration R reçue par le capteur 7, la superposition des images des différents traits lumineux forme l'image scanographique recherchée.

REVENDICATIONS

1.- Procédé pour la réalisation d'images tomographiques au moyen d'un appareil scanographique comportant un bâti rotatif (1) tournant autour de l'objet ou du corps à observer, une source rayonnements (6) solidaire en rotation dudit bâti (1) et au moins un capteur (7) recevant le faisceau d'exploration R émis par ladite source (6) après la traversée dudit objet ou dudit corps à observer, ledit faisceau d'exploration R effectuant, outre un mouvement de rotation correspondant à celui dudit bâti, un mouvement de balayage alternatif dans un plan orthogonal à l'axe de rotation X-X' de ce bâti (1),
caractérisé en ce que l'on forme, à partir du signal reçu par ledit capteur (7), un trait lumineux (9 ou 9,11) dont l'intensité instantanée est uniforme le long dudit trait et correspond à la quantité instantanée de rayonnement R reçue par ledit capteur (7) et en ce que l'on fait effectuer audit trait lumineux (9) un mouvement semblable et synchrone de celui du faisceau d'exploration R, de sorte à balayer une pluralité de fois une zone d'enregistrement suivant une pluralité de directions tournant avec la rotation dudit bâti (1).

2.- Dispositif pour la mise en oeuvre du procédé selon la revendication 1, comportant un bâti rotatif (1) tournant autour de l'objet ou du corps à observer, une source rayonnements (6) solidaire en rotation dudit bâti (1) et au moins un capteur (7) recevant le faisceau d'exploration R émis par ladite source (6) après la traversée dudit objet ou dudit corps à observer, ledit faisceau d'exploration R effectuant, outre un mouvement de rotation correspondant à celui dudit bâti, un mouvement de balayage alternatif dans un plan orthogonal à l'axe de rotation X-X' de ce bâti (1), caractérisé en ce qu'il comporte un dispositif lumineux linéaire (9 ou 9,11) dont l'intensité est uniforme le long dudit dispositif et correspond à la quantité instantanée de rayonnement R reçue par le capteur (7), et des moyens de couplage imposant audit dispositif lumineux linéaire (9 ou 9,11) un mouvement semblable et synchrone de celui du faisceau d'exploration R, de sorte que ce dispositif lumineux linéaire balaie une pluralité de fois une zone d'enregistrement suivant une pluralité de directions tournant avec la rotation dudit bâti (1).

3.- Dispositif selon la revendication 2, caractérisé en ce que le dispositif lumineux linéaire est constitué, au moins en partie, par un alignement (9) d'éléments électroluminescents, tels que des diodes.

4.- Dispositif selon la revendication 3, caractérisé en ce que l'éclairement de tous les éléments électroluminescents est semblable et significatif de la quantité de rayonnement reçue par le capteur (7).

5.- Dispositif selon la revendication 3, caractérisé en ce que l'éclairement du dispositif lumineux est constant et l'on prévoit, entre le dispositif lumineux linéaire (9) et la zone d'enregistrement, un filtre à transmission variable commandée (11), dont le facteur de transmission instantané est représentatif de la quantité de rayonnement reçue par le capteur (7).

6.- Dispositif selon l'une quelconque des revendications 2 à 5, dans lequel le balayage alternatif du faisceau d'exploration est obtenu par voie mécanique, caractérisé en ce que le dispositif lumineux linéaire est mécaniquement rendu solidaire de la pièce (2,3) en mouvement produisant ce balayage.

7.- Dispositif selon l'une quelconque des revendications 2 à 5, dans lequel le balayage alternatif du faisceau d'exploration est obtenu par voie électrique, caractérisé en ce que le mouvement du dispositif lumineux est synchronisé électriquement sur celui dudit faisceau d'exploration.

8.- Dispositif selon l'une quelconque des revendications 2 à 7,
caractérisé en ce que la zone d'enregistrement est constituée par une pellicule photographique.

0076180

1/1

Fig.1

Fig.2

Fig.3